# EUROPEAN PATENT APPLICATION

(11) **EP 4 760 257 A1**
(43) Date of publication of application: **17.06.2026**
(21) Application number: 25222452.2
(22) Date of filing: 11.12.2025
(51) Int. Cl.: G01N 33/536, G01N 33/543

(54) **METHOD OF MEASURING TARGET SUBSTANCE, AND REAGENT THEREFOR**

(30) Priority: 13.12.2024 JP 2024218586; 04.11.2025 JP 2025185678
(71) Applicant: Canon Kabushiki Kaisha, Tokyo, 146-8501 (JP)
(72) Inventor: SHIMANO, Tsutomu, Tokyo (JP); ISHIOKA, Shuhei, Tokyo (JP); WAKISAKA, Risa, Tokyo (JP); KANAZAKI, Kengo, Tokyo (JP); OONUKI, Sayaka, Tokyo (JP); FUNAYAMA, Kiyotake, Tokyo (JP)
(74) Representative: WESER & Kollegen Patentanwälte PartmbB

(57) **Abstract**

Provided are a reagent and a measurement method each excellent in measurement reproducibility. The reagent is for measuring a target substance in a specimen, wherein the target substance is ferritin, and wherein the reagent includes a first reagent and a second reagent including a particle on which a ligand that specifically binds to the target substance is immobilized, and wherein, when a first mixing step of obtaining a first mixed solution, in which the specimen containing the target substance and the first reagent are mixed, and a second mixing step of obtaining a second mixed solution, in which the first mixed solution and the second reagent are mixed, after 280 seconds or more have elapsed from the first mixing step, are performed, an amount of change in absorbance of the second mixed solution within 50 seconds from the mixing in the second mixing step is 0.15 or more.

## Description

### TECHNICAL FIELD

The present disclosure relates to a method of measuring a target substance, and a reagent therefor.

### BACKGROUND

There is given immunoturbidimetry using a particle as a simple and rapid immunological testing method. In this method, a dispersion of a latex particle to which a ligand having affinity to a target substance is bonded, and a specimen that may contain the target substance are mixed. At this time, the particle causes an aggregation reaction in accordance with the amount of the target substance in the specimen, and hence the target substance can be qualitatively or quantitatively determined by optically detecting the aggregation reaction as a variation in, for example, scattered light intensity, transmitted light intensity, or absorbance.

A measuring reagent for ferritin by immunoturbidimetry using a particle is disclosed in "Fundamental Performance of "latro Ferritin" Reagent for Measurement of Ferritin on a Multi-purpose Automated Analyzer," JJCLA Vol. 31, No. 5, 2006, and "Examination of "LZ Test Eiken FER" Reagent for Measurement of Ferritin for a Multi-purpose Automated Analyzer," Instruments and Reagents 26(4), 2003.

### SUMMARY

The reagent disclosed in each of "Fundamental Performance of "latro Ferritin" Reagent for Measurement of Ferritin on a Multi-purpose Automated Analyzer," JJCLA Vol. 31, No. 5, 2006, and "Examination of "LZ Test Eiken FER" Reagent for Measurement of Ferritin for a Multi-purpose Automated Analyzer," Instruments and Reagents 26(4), 2003, has had room for improvement in measurement reproducibility. The present disclosure is directed to providing a reagent and a measurement method each excellent in measurement reproducibility.

The present disclosure in its first aspect provides a reagent for measuring a target substance in a specimen, wherein the target substance is ferritin, wherein the reagent includes a first reagent including a buffering agent, and a second reagent including a particle on which a ligand that specifically binds to the target substance is immobilized, wherein, when a first mixing step of obtaining a first mixed solution, in which the specimen containing 1,000 ng/mL of the target substance and the first reagent are mixed at a volume ratio "specimen:first reagent" of 15:60, and a second mixing step of obtaining a second mixed solution, in which the first mixed solution and the second reagent are mixed at a volume ratio "first mixed solution:second reagent" of 75:30, after 280 seconds or more have elapsed from the mixing in the first mixing step are performed, an amount of change in absorbance of the second mixed solution within 50 seconds from the mixing in the second mixing step is 0.15 or more as specified in claim 1. Optional features are specified in claims 2 to 19.

In addition, the present disclosure in its second aspect provides a reagent for measuring a target substance in a specimen, wherein the target substance is a protein forming a complex that is hexameric or higher, wherein the reagent includes a first reagent including a buffering agent, and a second reagent including a particle on which a ligand that specifically binds to the target substance is immobilized, wherein, when a first mixing step of obtaining a first mixed solution, in which the specimen containing 1,000 ng/mL of the target substance and the first reagent are mixed at a volume ratio "specimen:first reagent" of 15:60, and a second mixing step of obtaining a second mixed solution, in which the first mixed solution and the second reagent are mixed at a volume ratio "first mixed solution:second reagent" of 75:30, after 280 seconds or more have elapsed from the mixing in the first mixing step are performed, an amount of change in absorbance of the second mixed solution within 50 seconds from the mixing in the second mixing step is 0.15 or more as specified in claim 20. Optional features are specified in claims 21 and 22.

Further, the present disclosure in its third aspect provides a measurement method for measuring a target substance in a specimen, the target substance being ferritin, the method including: a first mixing step of obtaining a first mixed solution in which the specimen and a first reagent are mixed; a second mixing step of obtaining a second mixed solution in which the first mixed solution and a second reagent including a particle on which a ligand that specifically binds to the target substance is immobilized are mixed; and a measurement step of measuring an absorbance of the second mixed solution, the specimen containing 500 ng/mL or more and 1,000 ng/mL or less of the target substance, an amount of change in absorbance of the second mixed solution within 50 seconds from the mixing in the second mixing step being 0.15 or more as specified in claim 23. Optional features are specified in claim 24.

Finally, the present disclosure in its fourth aspect provides a measurement method for measuring a target substance in a specimen, the target substance being a protein forming a complex that is hexameric or higher, the method including: a first mixing step of obtaining a first mixed solution in which the specimen and a first reagent are mixed; a second mixing step of obtaining a second mixed solution in which the first mixed solution and a second reagent including a particle on which a ligand that specifically binds to the target substance is immobilized are mixed; and a measurement step of measuring an absorbance of the second mixed solution, the specimen containing 500 ng/mL or more and 1,000 ng/mL or less of the target substance, an amount of change in absorbance of the second mixed solution within 50 seconds from the mixing in the second mixing step being 0.15 or more as specified in claim 25.

According to the present disclosure, the reagent and the measurement method each excellent in measurement reproducibility can be provided.

Features of the present disclosure will become apparent from the following description of embodiments. The following description of embodiments is described by way of example.

### DESCRIPTION OF THE EMBODIMENTS

Embodiments of the present disclosure are described in detail below. However, the technical scope of the present disclosure is not limited to the embodiments. A particle on which a ligand that specifically binds to a target substance is immobilized is hereinafter also referred to as "affinity particle," and a particle before the ligand that specifically binds to the target substance is immobilized thereon is also referred to as "pre-sensitization particle."

Some target substances have a plurality of ligand recognition sites. For example, ferritin is a complex of 24 proteins and may have 24 antibody recognition sites. The inventors have repeated intensive investigations in order to further improve measurement accuracy for the target substance having a plurality of antibody recognition sites such as ferritin, and as a result, have found that it is important to control a particle aggregation speed. Description is made below by taking ferritin as a specific example, but the effect of the present disclosure may be described by the same idea as long as a protein forming a complex that is hexameric or higher is adopted.

When ferritin is detected as a target substance by immunoturbidimetry using a particle, more than two particles may aggregate per target substance. Accordingly, aggregation patterns are diverse, and in some cases, multiparticle aggregation of three or more particles occurs, and in other cases, a plurality of two-particle aggregations occur. The amount of change in absorbance in the immunoturbidimetry using a particle depends on a change in particle diameter caused by aggregation, and hence when such a difference in aggregation state as described above occurs, the amount of change in absorbance may vary even with the same amount of ferritin. As a result, it has been conceived that fluctuations in measured values may occur even when a specimen containing the same amount of ferritin is measured.

Based on the above-mentioned idea, as a result of detailed analysis of particle aggregation behavior, it has been found that, as the aggregation speed at the time of the mixing of the specimen and the affinity particle, that is, the speed of the change in absorbance becomes higher, the reproducibility of the measured value becomes higher.

When the particle aggregation speed is high, a second particle rapidly aggregates with a first particle with which ferritin has reacted. This reaction occurs simultaneously throughout the entire reaction field, and hence it is conceived that uniform two-particle aggregation is easily formed. Meanwhile, when the particle aggregation speed is low, it takes time for the second particle to aggregate with the first particle with which ferritin has reacted. Accordingly, it is conceived that the proportion of aggregation by involvement in other aggregates already formed increases, and as a result, non-uniform aggregation is easily formed.

From the above-mentioned idea, the present disclosure is directed to a reagent for measuring a target substance in a specimen, wherein the target substance is ferritin, wherein the reagent includes a first reagent including a buffering agent, and a second reagent including a particle on which a ligand that specifically binds to the target substance is immobilized, and wherein, when a first mixing step of obtaining a first mixed solution, in which the specimen containing 1,000 ng/mL of the target substance and the first reagent are mixed at a volume ratio "specimen:first reagent" of 15:60, and a second mixing step of obtaining a second mixed solution, in which the first mixed solution and the second reagent are mixed at a volume ratio "first mixed solution:second reagent" of 75:30, after 280 seconds or more have elapsed from the mixing in the first mixing step are performed, an amount of change in absorbance of the second mixed solution within 50 seconds from the mixing in the second mixing step is 0.15 or more.

In this embodiment, the mixing of the specimen and the first reagent may refer to the addition of one of the specimen or the first reagent to the other, or may refer to the addition of the specimen and the first reagent to each other. The same applies to any other mixing in the present specification. In addition, the phrase "from the mixing" in this embodiment means from the time of the completion of stirring when the entire amount of the two kinds of liquids is mixed and the stirring is performed to sufficiently mix the two kinds of liquids. In addition, a time period from the time of the start of the mixing of the two kinds of liquids to the time of the completion of the stirring described above is 5 seconds or less. The stirring may be performed with a stirring bar, by irradiation with ultrasonic waves, or by tapping. For example, the fact that the amount of change in absorbance of the second mixed solution within 50 seconds from the mixing is 0.15 or more means that a difference between absorbances measured at two times different to each other within 50 seconds from the time of the completion of the stirring may be 0.15 or more. For example, a difference between an absorbance measured at the time of the completion of the stirring and an absorbance measured at the time when 50 seconds have elapsed from the time of the completion of the stirring may be 0.15 or more, or a difference between an absorbance measured at the time when 1 second has elapsed from the time of the completion of the stirring and an absorbance measured at the time when 42 seconds have elapsed from the time of the completion of the stirring may be 0.15 or more. For the term "mixing", the same applies to any other mixing in the present specification.

When the concentration of the specimen in the second mixed solution is less than 4.0 vol%, sufficient detection sensitivity cannot be obtained. When the concentration is more than 30.0 vol%, the particle aggregation is affected by components other than the target substance in the specimen. Thus, there is a risk in that measurement accuracy deteriorates and sufficient detection sensitivity cannot be obtained. In addition, the measurement accuracy also deteriorates when the concentration of the target substance in the specimen is high. The concentration of the specimen in the second mixed solution is more preferably 7.0 vol% or more and 30.0 vol% or less, still more preferably 10.0 vol% or more and 20.0 vol% or less.

In the measurement described above, the amount of change in absorbance within 50 seconds after the mixing of the first mixed solution and the second reagent at a volume ratio "first mixed solution:second reagent" of 75:30 is also referred to as "amount of change in absorbance at the initial stage of a reaction."

The fact that the amount of change in absorbance at the initial stage of a reaction is 0.15 or more means that the aggregation speed is high when the antigen and the affinity particle are mixed. Accordingly, excellent measurement reproducibility can be obtained when the amount of change in absorbance at the initial stage of a reaction is 0.15 or more. In particular, in the case where the ferritin concentration is 100 ng/mL or more, the ratio of the number of antigens to that of particles is large, and hence aggregation of a plurality of particles is likely to occur, and the effect of the present disclosure is great. The amount of change in absorbance at the initial stage of a reaction is more preferably 0.20 or more and 0.50 or less. The amount of change in absorbance exceeding 0.50 is not preferred because non-specific aggregation other than aggregation due to an antigen-antibody reaction may occur.

In addition, in the measurement described above, the amount of change in absorbance between 50 seconds and 250 seconds after the mixing of the first mixed solution and the second reagent at a volume ratio "first mixed solution:second reagent" of 75:30 is preferably 0.20 or more. The fact that the amount of change in absorbance is 0.20 or more means that the particle aggregation appropriately proceeds even in the second half of the aggregation reaction. Accordingly, a measurement result excellent in accuracy can be obtained. The amount of change in absorbance between 50 seconds and 250 seconds after the mixing is more preferably 0.25 or more and 0.80 or less.

### <Detection Method and Measurement Method>

A measurement method to be used in the present disclosure is immunoturbidimetry using a particle. The immunoturbidimetry using a particle is a method of optically detecting inter-particle aggregation that occurs when the affinity particle of the present disclosure and the specimen are mixed. In the present disclosure, an absorbance is used as a method of detecting the optical change. There is no limitation on an instrument used for the measurement, and any optical instrument capable of detecting the absorbance may be used. Of those, a general-purpose automatic analyzer, which is simple to control the dispensing amount of the specimen or the reagent, a mixing time, a measurement wavelength, and the like, is preferably used.

The measurement method of this embodiment is a measurement method for measuring a target substance in a specimen, the target substance being ferritin, the method including: a first mixing step of obtaining a first mixed solution in which the specimen and a first reagent are mixed; a second mixing step of obtaining a second mixed solution in which the first mixed solution and a second reagent including a particle on which a ligand that specifically binds to the target substance is immobilized are mixed; and a measurement step of measuring an absorbance of the second mixed solution, the specimen containing 500 ng/mL or more and 1,000 ng/mL or less of the target substance, an amount of change in absorbance of the second mixed solution within 50 seconds from the mixing in the second mixing step being 0.15 or more.

An example of the measurement method using the automatic analyzer is described below. First, 8 µL to 25 µL of the specimen is dispensed into a reaction container. Next, as the first mixing step, 50 µL to 150 µL of the first reagent is dispensed into the same reaction container, and after stirring, the temperature is adjusted to a predetermined temperature, and the container is kept warm for from 180 seconds to 600 seconds. The temperature at this time preferably falls within the range of from 20°C to 50°C.

After that, as the second mixing step, 10 µL to 150 µL of the second reagent is dispensed into the same reaction container, and after stirring, a reaction is performed for from 180 seconds to 600 seconds. At this time, it is preferred to start the second mixing step after 280 seconds or more have elapsed from the mixing in the first mixing step. When 280 seconds elapse from the mixing in the first mixing step, the specimen and the first reagent are uniformly mixed, and hence the particle aggregation becomes uniform and the measurement reproducibility improves. In addition, the reaction time of the second mixing step is more preferably 200 seconds or more and 600 seconds or less.

Further, the absorbance of the second mixed solution is measured, and the amount of change in absorbance is calculated. At this time, the absorbance 42 seconds after the mixing in the second mixing step is preferably 0.90 or more and 2.00 or less. This is because the absorbance 42 seconds after the mixing in the second mixing step indicates the absorbance at the starting point before the aggregation reaction proceeds, and when the value falls within the above-mentioned range, excellent measurement reproducibility can be obtained. In addition, the measurement wavelength of the absorbance preferably includes 500 nm or more and 750 nm or less.

The concentration of the affinity particle in the second mixed solution is preferably 0.02 mass/vol% or more and 0.10 mass/vol% or less. When the concentration is 0.02 mass/vol% or more and 0.10 mass/vol% or less, the particle aggregation easily becomes uniform, and excellent measurement reproducibility is obtained. The pH in the second mixed solution is preferably 5.0 or more and 11.0 or less.

The viscosity of the second mixed solution is preferably 0.95 mPa·s or more and 1.40 mPa·s or less. When the viscosity falls within the above-mentioned range, the particle aggregation speed is suppressed, and higher measurement accuracy is obtained. In addition, excellent measurement accuracy is obtained even when the concentration of the target substance is high.

### <Specimen and Target Substance>

The specimen that may be used in the present disclosure is not particularly limited as long as the specimen contains the target substance, and examples thereof include blood, serum, and plasma.

The target substance of the present disclosure is a protein having a molecular weight of 150 kDa or more and 500 kDa or less, or a protein forming a complex that is hexameric or higher. In addition, the target substance is more preferably a protein having a molecular weight of 300 kDa or more and 600 kDa or less. The molecular weight of ferritin given as an example in the present disclosure is 445 kDa, and the molecular weight is large. Thus, ferritin easily reacts with the antibody on a surface of the particle, and the effect of the present disclosure is easily obtained. In this embodiment, the specimen preferably contains 500 ng/mL or more and 1,000 ng/mL or less of the target substance.

### <First Reagent and Second Reagent>

The reagent of the present disclosure includes a first reagent including a buffering agent, and a second reagent including an affinity particle. The first reagent is used for the role of diluting the specimen or subjecting the specimen to prevent non-specific reaction. Accordingly, the first reagent may include a buffering agent, a sugar, a surfactant, a sensitizer, or a non-specific reaction inhibitor as described later.

The surface tension of the first reagent is preferably 20 mN/m or more and 50 mN/m or less. When the surface tension falls within the above-mentioned range, the affinity particle and the specimen are uniformly mixed. As a result, excellent measurement reproducibility is obtained.

The electrical conductivity of the first reagent is preferably 0.5 mS/cm or more and 70.0 mS/cm or less, more preferably 10.0 mS/cm or more and 70.0 mS/cm or less. When the electrical conductivity falls within the above-mentioned ranges, the electrostatic repulsion force of the affinity particle is maintained, and hence an interaction caused by an anionic specimen component, in particular, is uniformized for each affinity particle, and more excellent measurement reproducibility is obtained. The electrical conductivity of the first reagent is more preferably 0.5 mS/cm or more and 65.0 mS/cm or less.

The pH of each of the first reagent and the second reagent is preferably 5.0 or more and 11.0 or less. When the pH falls within the above-mentioned range, the dispersed state of the affinity particle and the mixed state when the specimen is mixed can be uniformized. Accordingly, more excellent measurement reproducibility is obtained. The pHs of the first reagent and the second reagent may be values different from each other.

It is preferred that the first reagent and the second reagent each include a buffering agent (buffer). The kind of the buffer is not particularly limited, and may be a substance from which a buffering capacity is obtained. For example, acetate, citrate, phosphate, Tris, glycine, borate, and carbonate buffers, and Good's buffers, such as MES, Bis-Tris, ADA, PIPES, ACES, MOPSO, BES, MOPS, TES, HEPES, TAPSO, POPSO, HEPSO, EPPS, Tricine, Bicine, TAPS, CHES, and CAPS, are suitably used. The buffering agents may be used alone or in combination thereof. In addition, the buffering agents used for the first reagent and the second reagent may be identical to or different from each other.

It is preferred that the first reagent and the second reagent each further include a sugar or a sugar alcohol. When the first reagent and the second reagent each include a sugar, the hydration of the surface of the affinity particle or the specimen component is promoted, an interaction between the affinity particle and the specimen component is reduced, and the measurement reproducibility improves. Examples of such sugar and sugar alcohol include, but are not limited to: monosaccharides, such as glucose and fructose; disaccharides, such as sucrose, lactose, maltose, cellobiose, and trehalose; or oligosaccharides, such as maltotriose and dextran; and sugar alcohols, such as erythritol, mannitol, sorbitol, and xylitol. The sugars and the sugar alcohols may each be used alone or in combination thereof.

It is preferred that the first reagent and the second reagent each further include a surfactant. As the surfactant, a known nonionic surfactant, anionic surfactant, cationic surfactant, or amphoteric surfactant may be used. It is preferred that the first reagent and the second reagent each include one or more of a sorbitan fatty acid ester, a polyoxyethylene alkyl ether, and a polyoxyethylene phenyl ether out of those surfactants. Those surfactants each have high hydrophilicity and high solubilizing and stabilizing effects on the specimen component. Accordingly, the interaction between the affinity particle and the specimen component is reduced, and excellent measurement reproducibility is obtained. The concentration of the surfactant is preferably 0.001 mass% or more and 0.200 mass% or less in the second mixed solution.

The first reagent may further include a sensitizer. Examples of the sensitizer include water-soluble polymers, such as polyethylene glycol, carboxymethyl cellulose, methyl cellulose, polyvinylpyrrolidone, polyvinyl alcohol, polyacrylic acid, and polyglycosyl ethyl methacrylate.

In addition, the first reagent and the second reagent of the present disclosure may each include a chelating agent, such as EDTA, CyDTA, DTPA, EGTA, NTA, or NTP, a protein, such as bovine serum albumin, casein, or gelatin, a non-specific reaction inhibitor such as a protein degradation product, an amino acid, animal serum, an antibody, an antibody fragment, or a reducing agent, a stabilizer, such as a protein or a preservative, or an inorganic salt, such as sodium chloride, potassium chloride, or calcium chloride.

### <Affinity Particle and Pre-sensitization Particle>

The zeta potential of the affinity particle of the present disclosure is preferably -50 mV or more and -15 mV or less. When the zeta potential is -50 mV or more and -15 mV or less, the balance between the electrostatic repulsion force and electrostatic attraction force of the affinity particle is appropriate. Accordingly, the dispersion of the affinity particle is stabilized, and more excellent measurement reproducibility is obtained. The zeta potential of the affinity particle is more preferably -40 mV or more and -15 mV or less.

The volume average particle diameter of the affinity particle of the present disclosure is preferably 200 nm or more and 500 nm or less. When the volume average particle diameter falls within the above-mentioned range, the particle number concentration falls within an appropriate range. Accordingly, the action of the specimen component on the affinity particle becomes more uniform, and excellent measurement reproducibility is obtained. The volume average particle diameter is more preferably 250 nm or more and 400 nm or less.

Further, the affinity particle of the present disclosure is an affinity particle obtained by mixing two kinds of affinity particles having different volume average particle diameters, and a difference in volume average particle diameter between the two kinds of affinity particles is preferably 50 nm or more and 250 nm or less. By mixing the two kinds of affinity particles having different volume average particle diameters, excellent measurement accuracy is obtained for a wider range of target substance concentrations. At that time, when the difference in particle diameter falls within the above-mentioned range, the action of the specimen component on the affinity particle is less likely to be biased, and hence excellent measurement accuracy is obtained over a wide range of target substance concentrations.

In addition, the refractive index of the affinity particle of the present disclosure is preferably 1.600 or more and 1.800 or less. Further, the affinity particle is more preferably an affinity particle obtained by mixing two kinds of affinity particles having different refractive indices, and a more excellent measurement range is obtained.

In addition, the protein amount with respect to the pre-sensitization particle in the affinity particle of the present disclosure is preferably 200 µg or less per 1 mg of the particle. This range means that the amount of albumin or the like as a stabilizer for the affinity particle is extremely small. Accordingly, excellent sensitivity is obtained.

As the pre-sensitization particle used in the present disclosure, a conventionally known particle may be used. For example, polystyrene, a styrene-butadiene copolymer, a styrene-styrenesulfonate copolymer, or a styrene-glycidyl methacrylate copolymer may be used. Of those, a particle containing a polymer having a repeating unit represented by the following formula (1) is preferred as the pre-sensitization particle: 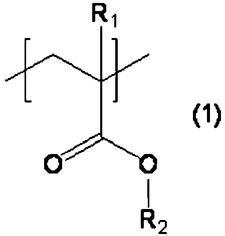 where R₁ represents a methyl group or a hydrogen atom, and R₂ represents a group having any one of an epoxy group, a hydroxy group, or a carboxy group.

When the pre-sensitization particle contains a polymer having the repeating unit represented by the formula (1), the surface of the affinity particle is easily hydrated, and the ionicity is low, and hence the influence of a hydrophobic ion component is suppressed. As a result, higher measurement accuracy is obtained. The formula (1) represents more preferably a structure represented by the following formula (1-A): 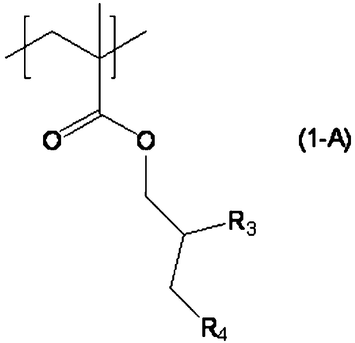 where at least one of R₃ or R₄ represents a hydroxy group, and the other thereof represents a hydroxy group or a group represented by the following formula (1-B): 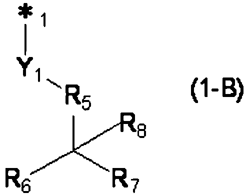 where R₅ represents a single bond or a methylene group, R₆, R₇, and R₈ are each selected from a hydrogen atom, a methyl group, a hydroxy group, a carboxy group, a hydroxymethyl group, and a carboxymethyl group, and at least one of R₆, R₇, or R₈ includes a hydroxy group or a carboxy group, Y₁ represents a sulfur atom or an imino group, and *₁ represents a bonding position with the structure represented by the formula (1-A).

Specific examples of the structure represented by the formula (1-A) are shown in the following formulae (1-A-1) to (1-A-12), but the present disclosure is not limited thereto.

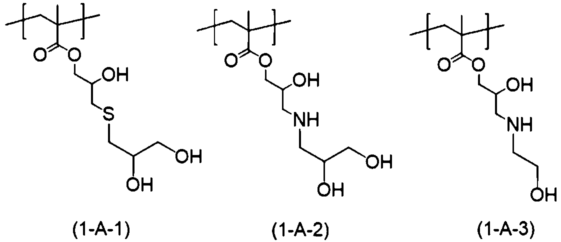

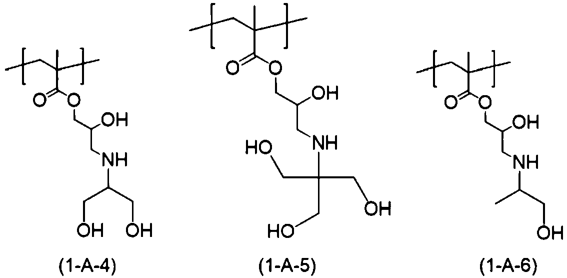

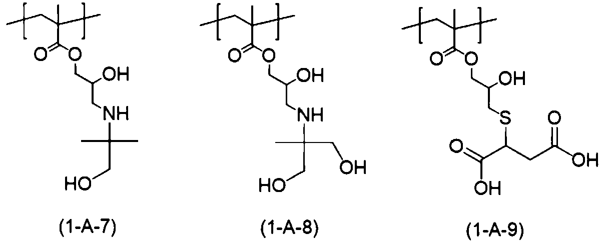

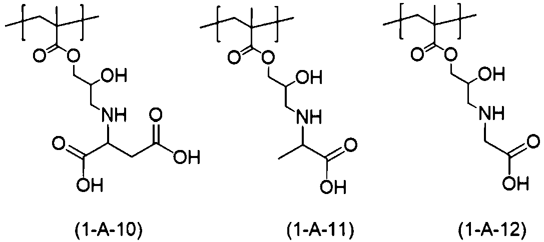

As a method of providing the particles having those structures, for example, glycidyl methacrylate is used. Specifically, a particle having the structure represented by the formula (1) can be obtained by using glycidyl methacrylate. In addition, a particle having the structure represented by the formula (1-A) can be obtained by bonding a compound having a mercapto group or a compound having an amino group to the epoxy group of glycidyl methacrylate.

In addition, the pre-sensitization particle preferably has a nitrogen-containing functional group, and more preferably contains a compound having an amidine group represented by the following formula (2). The amidine group suppresses the electrostatic repulsion force of the particle and increases the aggregation speed, and hence uniform two-particle aggregation is likely to occur, and excellent measurement reproducibility is obtained.

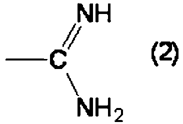

As a method of providing a particle having an amidine group, there is given, for example, a method involving using an initiator having an amidine group at the time of particle production. An example of the initiator having an amidine group is V-50 (2,2'-azobis(2-methylpropionamidine) dihydrochloride).

In addition, the pre-sensitization particle is preferably a particle containing a polymer having a repeating unit represented by the following formula (3): 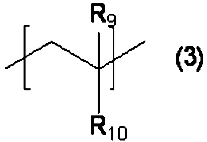 where R₉ represents a hydrogen atom or a methyl group, R₁₀ represents a substituted or unsubstituted phenyl group or naphthyl group, and each substituent thereof is a methyl group or an ethyl group, and the formula (3) may have two or more kinds of structures.

The structures represented by the formula (1), the formula (1-A), the formula (2), and the formula (3) may be included in the pre-sensitization particle or the affinity particle. In addition, the affinity particle preferably has the structure represented by the formula (4) or the formula (5), or both the structures represented by the formula (4) and the formula (5): 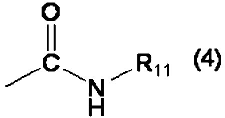 where R₁₁ represents a functional group having one or more hydroxy groups; and 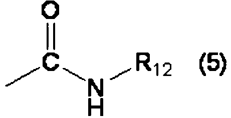 where R₁₂ represents a functional group having at least a carboxy group.

When the affinity particle has the structure represented by the formula (4) or the formula (5), the hydrophilicity of the particle can be increased. In addition, a state in which the electrostatic repulsion force is not increased can be maintained, and hence excellent measurement reproducibility and accuracy can be obtained.

As a method of providing the particle with the structure represented by the formula (4), there is given, for example, a method involving using an amino alcohol as a reaction terminator to be used at the time of the production of the affinity particle. As a method of providing the particle with the structure represented by the formula (5), there is given, for example, a method involving using an amino acid as a reaction terminator to be used at the time of the production of the affinity particle.

### <Ligand that specifically binds to Protein>

The ligand is a compound that specifically binds to a receptor of a specific target substance. A site where the ligand binds to the target substance is fixed, and has selectively or specifically high affinity. Examples thereof include an antigen and an antibody, an enzyme protein and a substrate therefor, a signal substance, such as a hormone or a neurotransmitter, and a receptor therefor, a nucleic acid, and avidin and biotin, but the present disclosure is not limited thereto to the extent that the object of the present disclosure can be achieved. Specific examples of the ligand include an antigen, an antibody, an antigen-binding fragment (e.g., Fab, F(ab')₂, F(ab'), Fv, or scFv), a naturally derived nucleic acid, an artificial nucleic acid, an aptamer, a peptide aptamer, an oligopeptide, an enzyme, and a coenzyme.

In this embodiment, the ligand is preferably an antibody or an antigen. Of those, an antibody having an isoelectric point of 5.0 or more and 8.0 or less is more preferred. The affinity particle using an antibody having an isoelectric point that falls within the range has a small charge on the surface of the affinity particle because of the influence of the antibody. As a result, an interaction between the affinity particle and the cationic component in the specimen is reduced, and excellent measurement accuracy is obtained. The isoelectric point may be measured by isoelectric focusing.

In the present disclosure, a method of immobilizing the ligand on the pre-sensitization particle may be performed by any known method, and the ligand can be immobilized by physically or chemically bonding the ligand to the pre-sensitization particle. For example, the ligand can be immobilized on the pre-sensitization particle by a covalent bond, a hydrogen bond, an ionic bond, electrostatic attraction, or a Van der Waals force. Examples of a method for the chemical bonding include a method involving utilizing a carbodiimide-mediated reaction or an NHS ester activation reaction, and a method of bonding a biotin-modified ligand to an avidin-bonded carboxy group.

The amount of the ligand with respect to 1.0 mg of the pre-sensitization particle is preferably 1.0 µg or more and 150.0 µg or less, more preferably 2.0 µg or more and 100.0 µg or less. Further, the affinity particle in the present disclosure is an affinity particle obtained by mixing two kinds of affinity particles, and it is still more preferred that the amounts of the ligand in the two kinds of affinity particles be different from each other.

In addition, the amount of the ligand in the second mixed solution is preferably 0.01 mg/mL or more and 2.00 mg/mL or less.

Examples of methods of measuring physical properties in the present disclosure are described below.

### <Method of measuring Viscosity>

The viscosity is measured with RE-85L (Toki Sangyo Co., Ltd.). 1.1 mL of the sample was loaded into a measurement container, and measured with a cone rotor at a shear rate of 100 rpm. The measurement temperature was set to 25°C by connecting a circulating constant temperature bath.

### <Method of measuring Surface Tension>

The surface tension is measured with an automatic surface tensiometer DyneMaster (Kyowa Interface Science Co., Ltd.). The measurement is performed by a plate method using a platinum plate under a temperature condition of 25°C.

### <Method of measuring Electrical Conductivity>

The electrical conductivity is measured with an electrical conductivity meter AS710 (AS ONE Corporation). The measurement is performed under the conditions of a temperature of 23°C and a humidity of 50%.

### <Method of measuring Zeta Potential of Particle>

The zeta potential of the particle is measured with Zetasizer Nano ZS (Malvern Panalytical). The zeta potential in the present disclosure is measured under a state in which the particle is dispersed in a 0.01 N potassium hydroxide aqueous solution having a pH of 7.8 so that the concentration of the particle becomes 0.003 mass/vol%. The measurement conditions are 25°C, latex (n≈1.59) is selected as the refractive index of the particle, and pure water is selected as a solvent. The measurement is performed ten times, and the average value of the ten measured values is adopted as the zeta potential.

### <Method of measuring Volume Average Particle Diameter of Particle>

The volume average particle diameter of the particle is measured with Zetasizer Nano ZS (Malvern Panalytical). The volume average particle diameter in the present disclosure is measured under a state in which the particle is dispersed in ion-exchanged water so that the concentration of the particle becomes 0.003 mass/vol%. The measurement conditions are 25°C, latex (n≈1.59) is selected as the refractive index of the particle, and pure water is selected as a solvent. The measurement is performed ten times, and the average value of the ten measured values is adopted as the volume average particle diameter.

In addition, a difference in volume average particle diameter when two or more kinds of particles having different particle diameters are mixed may be determined by observation with a scanning electron microscope or the like. Specifically, 300 or more particles are photographed at a magnification of 50,000 times, and the particle diameters of the 300 particles are measured by a known image processing method. The volume of each particle is determined from the measured particle diameter, and a volume distribution is prepared. A difference in particle diameter between the two kinds of particles may be determined by calculating the difference using the particle diameter that forms a peak in the prepared volume distribution as the volume average particle diameter of each particle.

### <Method of measuring Refractive Index of Particle>

The refractive index of the particle may be measured with Abbemat (manufactured by Anton Paar). In the following Examples, specifically, the refractive index was measured under a state in which the particle dispersion was dispersed so that the concentration of the particle became 5 mass%. As the measurement conditions, a temperature of 25°C and a measurement wavelength of 589.3 nm were selected. The particle refractive index was calculated from the Lorentz-Lorenz equation using the measured refractive index value, the specific gravity of the dispersion medium, the refractive index, and the specific gravity of the particle.

### <Method of measuring Amount of Antibody with respect to Particle (Antibody Sensitization Amount of Affinity Particle)>

A method of measuring the antibody sensitization amount of the affinity particle in the present disclosure is described. The antibody sensitization amount of the affinity particle with respect to the particle was determined by protein quantification. Herein, the term "antibody sensitization amount with respect to the particle" means the amount of the bound or adsorbed antibody per 1 mg of the particle.

First, 7 mL of the liquid A of Protein Assay BCA Kit (FUJIFILM Wako Pure Chemical Corporation) and 140 µL of the liquid B thereof are mixed, and the prepared liquid is adopted as a liquid AB. Next, 25 µL (particle concentration: 0.1 mass/vol%) of a dispersion of the affinity particle is added to 200 µL of the liquid AB, and the mixture is incubated at 60°C for 30 minutes. After that, the resultant solution is centrifuged at 4°C and 20,400×g for 5 minutes, and 200 µL of the supernatant is loaded into a 96-well microwell with a pipette. A standard sample, in which an antibody is mixed in a 10 mM HEPES buffer at an arbitrary concentration (five points within a concentration range of from 0 µg/mL to 200 µg/mL), is also loaded into a different location in the microwell in an amount of 200 µL, and the absorbance of the supernatant at 562 nm is collectively measured with a microplate reader, and the antibody amount is calculated from the calibration curve of the standard sample. The antibody amount (µg/mg) with respect to the particle is determined by dividing the calculated antibody amount by the mass of the particle.

### [Examples]

The present disclosure is described in detail below by way of Examples, but the present disclosure is not limited to these Examples.

### (Synthesis of Particle 1)

The synthesis of a particle 1 includes the following steps 1 to 3.

(Step 1) 71.75 g of styrene (St: Kishida Chemical Co., Ltd.), 1.30 g of divinylbenzene (DVB: Kishida Chemical Co., Ltd.), and 1,190.67 g of ion-exchanged water were weighed into a 2 L four-necked separable flask to prepare a mixed solution. Oxygen was removed from the inside of the four-necked separable flask by holding the mixed solution at 70°C under stirring at 200 rpm and performing a nitrogen flow at a flow rate of 200 mL/min. Next, a dissolved solution of 3.11 g of V-50 (FUJIFILM Wako Pure Chemical Corporation) dissolved in 50 g of ion-exchanged water, which had been separately prepared, was added to the mixed solution, and a polymerization reaction was performed for 48 hours to provide a dispersion containing a copolymer particle of St and DVB.

(Step 2) Next, 148.29 g of the dispersion diluted with ion-exchanged water so as to have a solid content concentration of 2.0 mass% was weighed into another four-necked separable flask. Next, 0.39 g of glycidyl methacrylate (GMA: Kishida Chemical Co., Ltd.) was added thereto. Oxygen was removed from the inside of the four-necked separable flask by holding the mixed solution at 70°C under stirring at 100 rpm and performing a nitrogen flow at a flow rate of 200 mL/min. Then, a dissolved solution of 0.018 g of V-50 dissolved in 1 g of ion-exchanged water, which had been separately prepared, was added to the mixed solution. A dispersion containing a St/DVB/GMA composite particle was obtained by continuing stirring for 17 hours.

(Step 3) Finally, an aqueous solution in mercaptosuccinic acid (MSA: FUJIFILM Wako Pure Chemical Corporation) and 3-mercapto-1,2-propanediol (MPD: FUJIFILM Wako Pure Chemical Corporation) were dissolved, which had been prepared in advance, was added to the dispersion containing the St/DVB/GMA composite particle. At this time, the aqueous solution was prepared so that the molar ratio of 3-mercapto-1,2-propanediol to mercaptosuccinic acid was 6:4 (molar fraction), and the total number of moles of MSA and MPD was adjusted to be equal to the number of moles of the glycidyl methacrylate. Subsequently, triethylamine (Kishida Chemical Co., Ltd.) was added thereto to adjust the pH to 10. Next, the dispersion was heated to 70°C under stirring at 200 rpm, and the state was maintained for an additional 18 hours. Thus, a dispersion of a particle 1 was obtained. After that, the operation of separating the particle 1 from the dispersion with a centrifuge and redispersing the particle 1 in ion-exchanged water was repeated eight times to purify the particle 1, and finally, a particle 1 dispersion having a solid content concentration of 5.0 mass% was obtained. The volume average particle diameter of the obtained particle 1 was 400 nm.

### (Synthesis of Particle 2 to Particle 6)

A particle 2 to a particle 4 were each synthesized by the same experimental operation as that in the method of synthesizing the particle 1 except that, in the method of synthesizing the particle 1, the amount of styrene, the amount of DVB, the amount of V-50, and the stirring speed used in the step 1, the amount of GMA in the step 2, and the molar fractions of MSA and MPD in the step 3 were changed as shown in Table 1. Those particles each have the structure represented by the formula (1) derived from GMA on a surface of the particle. Further, those particles each have the structure represented by the formula (2), which is a nitrogen-containing compound derived from V-50. In addition, polystyrene particles IMMUTEX P0307 and IMMUTEX P0322 manufactured by JSR Corporation were prepared as a particle 5 and a particle 6. The physical properties of the obtained particle 1 to particle 6 are collectively shown in Table 1.

In each of the particles 1 to 6, the core particle included a styrene-divinylbenzene copolymer, and contained a polymer having the structural unit represented by the formula (1) on its surface. More specifically, the polymer had the structural unit represented by the formula (1) in which R₁ represented a methyl group, and R₂ was represented by the following formula (31), formula (32), formula (33), or formula (34): 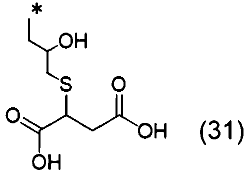 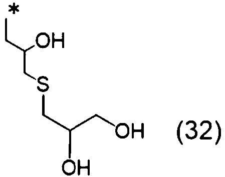 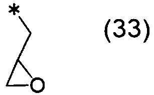 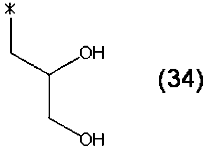 where * represents a bonding position with the structure represented by the formula (1).

**(Table 1)**

| | Synthesis conditions | | | | | Physical properties |
|---|---|---|---|---|---|---|
| | Step 1 | | | | Step 2 | |
| | Amount of styrene (g) | Amount of DVB (g) | Amount of V-50 (g) | Stirring speed (rpm) | Amount of GMA (g) | Volume average particle diameter (nm) |
| Particle 1 | 71.75 | 1.30 | 3.11 | 200 | 0.39 | 400 |
| Particle 2 | 43.05 | 0.78 | 1.87 | 200 | 0.39 | 350 |
| Particle 3 | 21.53 | 0.39 | 0.93 | 200 | 0.39 | 300 |
| Particle 4 | 21.53 | 0.39 | 0.93 | 140 | 1.54 | 250 |
| Particle 5 | "IMMUTEX P0307" manufactured by JSR Life Sciences, LLC | | | | | 430 |
| Particle 6 | "IMMUTEX P0322" manufactured by JSR Life Sciences, LLC | | | | | 290 |

### (Preparation of Affinity Particle 1)

300 µL (3 mg in terms of particle solid content) of the dispersion of the particle 1 diluted with ion-exchanged water to a solid content concentration of 1.0 mass/vol% was aliquoted into a 1.5 mL microtube. 90 µL of a 5.0 mass% aqueous solution of 1-(3-dimethylaminopropyl)-3-ethylcarbodiimide hydrochloride and 90 µL of a 5.0 mass% aqueous solution of N-hydroxysulfosuccinimide sodium salt were added thereto. The mixture was stirred at room temperature for 30 minutes to provide a dispersion of a particle having an activated carboxy group (activated particle dispersion).

After centrifugal washing, 270 µL of phosphate-buffered saline (hereinafter referred to as "PBS") having a pH of 5.5 was added thereto, and the particle having an activated carboxy group was dispersed with an ultrasonic wave.

24 µL (0.12 mg in terms of antibody amount) of a 5.0 mg/mL dispersion of a mouse monoclonal anti-ferritin antibody (isoelectric point: 7.1) was added as a ligand thereto, and the mixture was stirred at room temperature for 3 hours so that the particle was sensitized with the antibody. After that, 1.0 mL of a 1 mol/L tris(hydroxymethyl)aminomethane (Tris) solution having a pH of 8.0 was added as a reaction terminator thereto, and the mixture was allowed to stand overnight under an environment at 4°C to provide an antibody-sensitized test particle. After the centrifugal washing of the test particle, 500 µL of PBS was added thereto to provide an affinity particle 1.

The reaction is terminated by the bonding of an amino group in the reaction terminator to the activated carboxy group on the surface of the particle, and hence the surface of each affinity particle can be said to have a structure derived from the reaction terminator.

### (Preparation of Affinity Particle 2 to Affinity Particle 10)

An affinity particle 2 to an affinity particle 10 were each prepared by the same experimental operation as that in the preparation of the affinity particle 1 except that, in the method of preparing the affinity particle 1, the kind of the particle, the kind of the antibody, the addition amount of the antibody dispersion, and the kind and concentration of the reaction terminator were changed as shown in Table 2.

**(Table 2)**

| | Particle No. | Kind of antibody | Addition amount of antibody dispersi on (µL) | Amount of antibody with respect to 1 mg of pre-sensitizatio n particle (µg/mg) | Reaction terminator | Refractive index |
|---|---|---|---|---|---|---|
| Affinity particle 1 | 1 | Mouse monoclonal anti-ferritin antibody (isoelectric point: 7.1) | 24 | 40 | 1 mol/L Tris | 1.705 |
| Affinity particle 2 | 2 | Mouse monoclonal anti-ferritin antibody (isoelectric point: 7.1) | 24 | 40 | 1 mol/L Tris | 1.705 |
| Affinity particle 3 | 3 | Mouse monoclonal anti-ferritin antibody (isoelectric point: 7.1) | 24 | 40 | 1 mol/L Tris | 1.705 |
| Affinity particle 4 | 4 | Mouse monoclonal anti-ferritin antibody (isoelectric point: 7.1) | 6 | 10 | 1 mol/L Tris | 1.654 |
| Affinity particle 5 | 1 | Mouse monoclonal anti-ferritin antibody (isoelectric point: 7.1) | 24 | 40 | 0.3 mol/L Glycine | 1.705 |
| Affinity particle 6 | 1 | Mouse monoclonal anti-ferritin antibody (isoelectric point: 7.1) | 24 | 40 | None | 1.705 |
| Affinity particle 7 | 5 | Mouse monoclonal anti-ferritin antibody (isoelectric point: 7.1) | 24 | 25 | 1 mol/L Tris | 1.647 |
| Affinity particle 8 | 5 | Mouse monoclonal anti-ferritin antibody (isoelectric point: 7.1) | 10 | 10 | 1 mol/L Tris | 1.647 |
| Affinity particle 9 | 5 | Mouse monoclonal anti-ferritin antibody (isoelectric point: 7.1) | 24 | 25 | 1 mass% BSA | 1.647 |
| Affinity particle 10 | 6 | Mouse monoclonal anti-ferritin antibody (isoelectric point: 7.1) | 24 | 25 | 1 mol/L Tris | 1.647 |

### (Preparation of Second Reagent 1)

A second reagent 1 was prepared by using the prepared affinity particles 1 and 4, Specifically, the affinity particles 1 and 4 were each centrifuged, and re-dispersed in 500 µL of a buffer (HEPES buffer) in which 10 mM HEPES, 0.01 mass% polyoxyethylene nonylphenyl ether (Triton X-100: Kishida Chemical Co., Ltd.), and 10 mass% sucrose (viscosity modifier) were dissolved in ion-exchanged water. After that, the contents were mixed and diluted with the HEPES buffer so that the concentrations of the affinity particle 1 and the affinity particle 2 became 0.08 mass/vol% and 0.08 mass/vol%, respectively, and the total concentration was 0.16 mass/vol% to provide a second reagent 1.

### (Preparation of Second Reagent 2 to Second Reagent 11)

A second reagent 2 to a second reagent 11 were each prepared by the same experimental operation as that in the preparation of the second reagent 1 except that, in the preparation of the second reagent 1, the kind of the affinity particle, the concentration of the affinity particle, and the composition of the viscosity modifier added to the HEPES buffer were changed as shown in Table 3. The physical properties of the obtained second reagent 1 to second reagent 11 are collectively shown in Table 3.

**(Table 3)**

| | Preparation conditions | | | | |
|---|---|---|---|---|---|
| | Kind and concentration of affinity particle | | Viscosity modifier added to HEPES buffer | | Zeta potential (mV) |
| Second reagent 1 | Affinity particle 1 | : 0.08 mass/vol% | Sucrose | : 10 mass% | -25 |
| | Affinity particle 4 | : 0.08 mass/vol% | | | |
| Second reagent 2 | Affinity particle 2 | : 0.08 mass/vol% | Sucrose | : 10 mass% | -25 |
| | Affinity particle 4 | : 0.08 mass/vol% | | | |
| Second reagent 3 | Affinity particle 3 | : 0.08 mass/vol% | Sucrose | : 10 mass% | -25 |
| | Affinity particle 4 | : 0.08 mass/vol% | | | |
| Second reagent 4 | Affinity particle 1 | : 0.10 mass/vol% | Sucrose | : 10 mass% | -20 |
| Second reagent 5 | Affinity particle 7 | : 0.10 mass/vol% | Sucrose | : 10 mass% | -45 |
| Second reagent 6 | Affinity particle 5 | : 0.08 mass/vol% | Sucrose | : 10 mass% | -25 |
| | Affinity particle 4 | : 0.08 mass/vol% | | | |
| Second reagent 7 | Affinity particle 6 | : 0.08 mass/vol% | Sucrose | : 10 mass% | -30 |
| | Affinity particle 4 | : 0.08 mass/vol% | | | |
| Second reagent 8 | Affinity particle 1 | : 0.08 mass/vol% | None | | -25 |
| | Affinity particle 4 | : 0.08 mass/vol% | | | |
| Second reagent 9 | Affinity particle 9 | : 0.10 mass/vol% | Sucrose | : 10 mass% | -48 |
| Second reagent 10 | Affinity particle 10 | : 0.10 mass/vol% | Sucrose | : 10 mass% | -48 |
| Second reagent 11 | Affinity particle 8 | : 0.10 mass/vol% | Sucrose | : 10 mass% | -50 |

### (Preparation of First Reagent)

As shown in Table 4, a liquid in which each material was mixed in a predetermined amount in ion-exchanged water was used as a first reagent. The physical properties of the obtained first reagent are shown in Table 4.

**(Table 4)**

| | Kind and addition amount of material | Physical properties of first reagent | |
|---|---|---|---|
| | | Surface tension (mN/m) | Electrical conductivity (mS/cm) |
| First reagent | 50 mM HEPES | 35 | 18.6 |
| | 0.05 mass% Triton X-100 | | |
| | 1.0 mass% NaCl | | |

### (Measurement of Amount of Change in Absorbance)

A discrete clinical chemistry automatic analyzer (TBA-120FR: Canon Medical Systems Corporation) was used as a measuring device. A specimen containing 1,000 ng/mL of ferritin serving as a target substance was used, and assay parameters were set as follows: specimen: 15 µL; first reagent 1: 60 µL; second reagent: 30 µL; and measurement wavelength: 572 nm. The optical path length of the device is 5 mm, but an absorbance converted to that in the case where the optical path length is 1 cm is obtained as a measured value.

In the obtained reaction curve, the amount of change in absorbance between immediately after the dispensing of the second reagent and 50 seconds thereafter, and the amount of change in absorbance between 50 seconds and 250 seconds after the dispensing were calculated. The photometric points of the device were not set at the timings of 50 seconds and 250 seconds after the dispensing, and hence an approximate formula was calculated from the graph of the obtained reaction curve, and absorbances at 50 seconds and 250 seconds after the dispensing were calculated.

### (Creation of Calibration Curve)

First, standard solutions having ferritin concentrations of 0 ng/mL, 100 ng/mL, 250 ng/mL, 500 ng/mL, and 1,000 ng/mL were prepared. Those standard solutions were measured under the following assay parameter conditions: specimen: 15 µL; first reagent 1: 60 µL; second reagent: 30 µL; and measurement wavelength: 572 nm.

The value of a difference OD(33)-OD(19) between an absorbance OD(19) at photometric point 19 and an absorbance OD(33) at photometric point 33 in the obtained reaction curve was calculated as the amount of change in absorbance ΔOD. Next, a calibration curve showing a relationship between each ferritin concentration and ΔOD was created. Those experiments were performed for each second reagent used in each Example.

### (Evaluation Method for Measurement Reproducibility)

Through use of the same measuring device and assay parameters, the value of the difference OD(33)-OD(19) between the absorbance OD(19) at photometric point 19 and the absorbance OD(33) at photometric point 33 was calculated as the amount of change in absorbance ΔOD, and a ferritin concentration was quantified by using the calibration curve, which had been prepared in advance.

A specimen A containing 970 ng/mL of ferritin, a specimen B containing 550 ng/mL of ferritin, and a specimen C containing 106 ng/mL of ferritin were used as specimens, and were each measured five times. The coefficient of variation (CV) of the obtained ferritin concentration (hereinafter referred to as "CV") was calculated. The obtained CV was evaluated according to the following criteria.
A: The CV was less than 0.8.
B: The CV was 0.8 or more and less than 1.0.
C: The CV was 1.0 or more and less than 1.5.
D: The CV was 1.5 or more and less than 2.0.
E: The CV was 2.0 or more and less than 3.0.
F: The CV was 3.0 or more.

### (Method of evaluating Measurement Accuracy)

The average value of the ferritin concentrations when the specimen A containing 970 ng/mL of ferritin, the specimen B containing 550 ng/mL of ferritin, and the specimen C containing 106 ng/mL of ferritin were used and each measured five times by the same measurement method as the method of evaluating measurement reproducibility was calculated. The obtained average value of the ferritin concentrations was evaluated according to the following criteria.
A: Deviation between the obtained average value and the above-mentioned ferritin concentration was less than 3%.
B: Deviation between the obtained average value and the above-mentioned ferritin concentration was 3% or more and less than 5%.
C: Deviation between the obtained average value and the above-mentioned ferritin concentration was 5% or more and less than 10%.
D: Deviation between the obtained average value and the above-mentioned ferritin concentration was 10% or more and less than 15%.
E: Deviation between the obtained average value and the above-mentioned ferritin concentration was 15% or more.

### (Examples 1 to 8 and Comparative Examples 1 to 3)

The second reagent 1 to the second reagent 11 were evaluated. The obtained physical properties and evaluation results are collectively shown in Table 5 and Table 6.

**(Table 5)**

| | | Amount of change in absorbance 50 seconds after dispensing | Amount of change in absorbance between 50 seconds and 250 seconds after dispensing | Evaluation result for measurement reproducibility | | |
|---|---|---|---|---|---|---|
| | | | | CV value at 970 ng/mL | CV value at 550 ng/mL | CV value at 106 ng/mL |
| Example 1 | Second reagent 1 | 0.25 | 0.30 | A | A | A |
| Example 2 | Second reagent 2 | 0.20 | 0.30 | A | B | B |
| Example 3 | Second reagent 3 | 0.15 | 0.22 | B | B | C |
| Example 4 | Second reagent 4 | 0.25 | 0.15 | A | A | A |
| Example 5 | Second reagent 5 | 0.18 | 0.17 | C | C | C |
| Example 6 | Second reagent 6 | 0.25 | 0.30 | A | A | A |
| Example 7 | Second reagent 7 | 0.25 | 0.30 | B | B | C |
| Example 8 | Second reagent 8 | 0.28 | 0.30 | B | B | B |
| Comparative Example 1 | Second reagent 9 | 0.10 | 0.13 | E | E | D |
| Comparative Example 2 | Second reagent 10 | 0.13 | 0.14 | D | D | D |
| Comparative Example 3 | Second reagent 11 | 0.11 | 0.12 | D | D | D |

**(Table 6)**

| | | Evaluation result for measurement accuracy | | |
|---|---|---|---|---|
| | | CV value at 970 ng/mL | CV value at 550 ng/mL | CV value at 106 ng/mL |
| Example 1 | Second reagent 1 | A | A | A |
| Example 2 | Second reagent 2 | A | A | A |
| Example 3 | Second reagent 3 | A | A | B |
| Example 4 | Second reagent 4 | C | C | A |
| Example 5 | Second reagent 5 | C | C | C |
| Example 6 | Second reagent 6 | A | A | A |
| Example 7 | Second reagent 7 | B | A | B |
| Example 8 | Second reagent 8 | A | A | A |
| Comparative Example 1 | Second reagent 9 | D | C | C |
| Comparative Example 2 | Second reagent 10 | C | C | C |
| Comparative Example 3 | Second reagent 11 | D | C | C |

Those results show that that the reagent satisfying the provisions of the present disclosure provides excellent measurement reproducibility and accuracy over a wide range of ferritin concentrations.

Ferritin has been described as an example of the target substance that may be used in the present disclosure. A protein in which a plurality of subunits are associated such as ferritin may have a plurality of sites recognized by an antibody. Such protein may allow more than two particles to aggregate per target substance. Accordingly, aggregation patterns are diverse, and it is conceived that in some cases, multiparticle aggregation of three or more particles occurs, and in other cases, a plurality of two-particle aggregations occur. The amount of change in absorbance in immunoturbidimetry using a particle depends on a change in particle diameter caused by aggregation, and hence when such a difference in aggregation state as described above occurs, the amount of change in absorbance may vary even with the same amount of the target substance. As a result, a fluctuation may occur in the measured value even when a specimen containing the same amount of the target substance is measured. From this viewpoint, the measurement method of the present disclosure may be said to target a substance having a plurality of reaction sites with respect to a ligand that specifically binds to the target substance.

Various embodiments have been described in detail above but it will be understood that the present disclosure is not limited to these embodiments and encompasses all modifications, variants, alternatives and equivalents falling within the scope of the appended claims.

## Claims

1. A reagent for measuring a target substance in a specimen,
wherein the target substance is a protein forming a complex that is hexameric or higher,
wherein the reagent includes a first reagent including a buffering agent, and a second reagent including a particle on which a ligand that specifically binds to the target substance is immobilized, and
wherein, when a first mixing step of obtaining a first mixed solution, in which the specimen containing 1,000 ng/mL of the target substance and the first reagent are mixed at a volume ratio "specimen:first reagent" of 15:60, and a second mixing step of obtaining a second mixed solution, in which the first mixed solution and the second reagent are mixed at a volume ratio "first mixed solution:second reagent" of 75:30, after 280 seconds or more have elapsed from the mixing in the first mixing step are performed, an amount of change in absorbance of the second mixed solution within 50 seconds from the mixing in the second mixing step is 0.15 or more.

2. The reagent according to claim 1, wherein the target substance is a protein having a molecular weight of 300 kDa or more and 600 kDa or less.

3. The reagent according to claim 1 or 2, wherein the ligand is an antibody having an isoelectric point of 5.0 or more and 8.0 or less.

4. The reagent according to any one of claims 1 to 3, wherein the target substance is ferritin.

5. The reagent according to any one of claims 1 to 4, wherein the specimen is selected from the group consisting of blood, serum, and plasma each including the target substance.

6. The reagent according to any one of claims 1 to 5, wherein the amount of change in absorbance of the second mixed solution within 50 seconds after the mixing of the first mixed solution and the second reagent at a volume ratio "first mixed solution:second reagent" of 75:30 is 0.20 or more.

7. The reagent according to any one of claims 1 to 6, wherein the amount of change in absorbance of the second mixed solution between 50 seconds and 250 seconds after the mixing of the first mixed solution and the second reagent at a volume ratio "first mixed solution:second reagent" of 75:30 is 0.20 or more.

8. The reagent according to any one of claims 1 to 7, wherein a surface tension of the first reagent is 20 mN/m or more and 50 mN/m or less.

9. The reagent according to any one of claims 1 to 8, wherein the first reagent and the second reagent each include at least one of a sugar or a sugar alcohol.

10. The reagent according to claim 9, wherein the first reagent and the second reagent each include one or more surfactants selected from the group consisting of a sorbitan fatty acid ester, a polyoxyethylene alkyl ether, and a polyoxyethylene phenyl ether.

11. The reagent according to any one of claims 1 to 10, wherein an electrical conductivity of the first reagent is 0.5 mS/cm or more and 70.0 mS/cm or less.

12. The reagent according to any one of claims 1 to 11, wherein a zeta potential of the particle is -50 mV or more and -15 mV or less.

13. The reagent according to any one of claims 1 to 12, wherein the particle contains a polymer having a structure represented by the following formula (1): 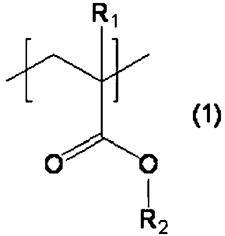 where R₁ represents a methyl group or a hydrogen atom, and R₂ represents a group having any one of an epoxy group, a hydroxy group, or a carboxy group.

14. The reagent according to claim 13, wherein the formula (1) represents a structure represented by the following formula (1-A): 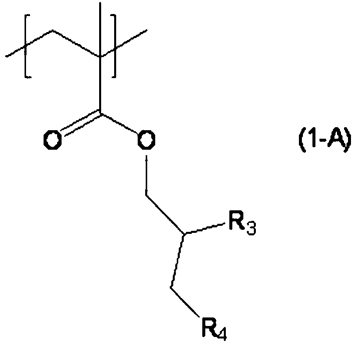 where at least one of R₃ or R₄ represents a hydroxy group, and another thereof represents a hydroxy group or a group represented by the following formula (1-B): 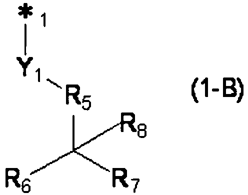 where R₅ represents a single bond or a methylene group, R₆, R₇, and R₈ are each selected from a hydrogen atom, a methyl group, a hydroxy group, a carboxy group, a hydroxymethyl group, and a carboxymethyl group, and at least one of R₆, R₇, or R₈ includes a hydroxy group or a carboxy group, Y₁ represents a sulfur atom or an imino group, and *₁ represents a bonding position with the structure represented by the formula (1-A).

15. The reagent according to any one of claims 1 to 14, wherein the particle has a nitrogen-containing functional group.

16. The reagent according to any one of claims 1 to 15, wherein the particle contains a compound having an amidine group represented by the following formula (2):

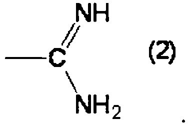

17. The reagent according to any one of claims 1 to 16, wherein the particle contains a polymer having a repeating unit represented by the following formula (3): 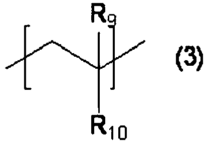 where R₉ represents a hydrogen atom or a methyl group, R₁₀ represents a substituted or unsubstituted phenyl group or naphthyl group, and each substituent thereof is a methyl group or an ethyl group, and the formula (3) may have two or more kinds of structures.

18. The reagent according to any one of claims 1 to 17, wherein the particle has a structure represented by the following formula (4): 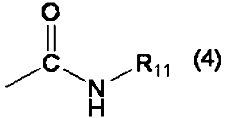 where R₁₁ represents a functional group having one or more hydroxy groups.

19. The reagent according to any one of claims 1 to 18, wherein the particle has a structure represented by the following formula (5): 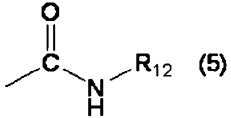 where R₁₂ represents a functional group having at least a carboxy group.

20. The reagent according to any one of claims 1 to 19, wherein a volume average particle diameter of the particle is 200 nm or more and 500 nm or less.

21. The reagent according to any one of claims 1 to 20, wherein the particle is a particle obtained by mixing two kinds of particles having different volume average particle diameters, and a difference in volume average particle diameter between the two kinds of particles is 250 nm or less.

22. The reagent according to any one of claims 1 to 21, wherein a measurement wavelength of the absorbance includes 500 nm or more and 750 nm or less.

23. A measurement method for measuring a target substance in a specimen, the method using the reagent of any one of claims 1 to 22,
the target substance being a protein forming a complex that is hexameric or higher,
the method comprising:
a first mixing step of obtaining a first mixed solution in which the specimen and the first reagent are mixed;
a second mixing step of obtaining a second mixed solution in which the first mixed solution and a second reagent including a particle on which a ligand that specifically binds to the target substance is immobilized are mixed; and
a measurement step of measuring an absorbance of the second mixed solution,
the specimen containing 500 ng/mL or more and 1,000 ng/mL or less of the target substance,
an amount of change in absorbance of the second mixed solution within 50 seconds from the mixing in the second mixing step being 0.15 or more.

24. The measurement method according to claim 23, wherein a concentration of the specimen in the second mixed solution is 7.0 vol% or more and 30.0 vol% or less.

25. The measurement method according to claim 23 or 24, wherein
the target substance is ferritin.
